# EUROPÄISCHE PATENTSCHRIFT

(11) **EP 2 347 002 B1**
(45) Veröffentlichungstag und Bekanntmachung des Hinweises auf die Patenterteilung: **07.05.2014**
(21) Anmeldenummer: 09771291.3
(22) Anmeldetag: 09.11.2009
(51) Int. Cl.: C12P 19/04, C12M 1/12, C12M 3/00

(54) **VERFAHREN ZUR MIKROBIELLEN HERSTELLUNG EINES CELLULOSE ENTHALTENDEN KÖRPERS**
PROCESS FOR THE MICROBIAL PRODUCTION OF A CELLULOSE CONTAINING BODY
PROCÉDÉ DE PRODUCTION MICROBIENNE D'UN CORPS CONTENANT DE LA CELLULOSE

(30) Priorität: 07.11.2008 DE 102008056413
(43) Veröffentlichungstag der Anmeldung: 27.07.2011
(73) Patentinhaber: Bioregeneration GmbH, 85748 Garching (DE)
(72) Erfinder: BERTHOLDT, Günter, 73092 Heiningen (DE); WEUSTER-BOTZ, Dirk, 85221 Dachau (DE); HOFINGER, Michael, 85408 Burgkirchen (DE); SEIFE, Katharina, 50679 Köln (DE)
(74) Vertreter: Keilitz, Wolfgang
(86) Internationale Anmeldenummer: PCT/EP2009/007998
(87) Internationale Veröffentlichungsnummer: WO 2010/052019

(56) Entgegenhaltungen:
- EP-A2- 0 396 344
- WO-A1-89/04865
- WO-A1-89/11529
- WO-A1-02/081725
- WO-A2-2008/040729

## Beschreibung

### Hintergrund der Erfindung

Die Erfindung betrifft ein Verfahren zur Herstellung eines Cellulose umfassenden Körpers. Schließlich betrifft die Erfindung eine Anordnung zur Herstellung eines Körpers aus Cellulose.

### Stand der Technik

Aus dem Stand der Technik ist es bekannt, dass Mikroorganismen Cellulose bilden können. Zum Beispiel synthetisiert das Bakterium *Gluconacetobacter xylinus* (früher als *Acetobacter xylinum* bezeichnet) in einer Standkultur an der Grenzschicht zwischen einem Nährmedium und der Luft reine Cellulose, die extrazellulär abgegeben wird. Diese sehr langkettigen Cellulosemoleküle lagern sich zu Bändern, sogenannten "Ribbons" zusammen, wobei sich durch Wasserstoffbrückenbindungen und Wasseranlagerungen hochkristalline Strukturen ausbilden. Diese Cellulose wird deshalb auch als mikrokristalline Cellulose bezeichnet. Die Bänder verfilzen miteinander und bilden makroskopisch eine feste Schicht von gel- bis knorpelartiger Konsistenz.

Es konnte gezeigt werden, dass diese mikrokristalline Cellulose bakteriellen Ursprungs in Säuretiergewebe nicht abgebaut wird und auch keine Fremdkörperreaktionen hervorruft. Auch wurden bereits mit zufriedenstellenden Ergebnissen Hohlkörper aus mikrokristalliner Cellulose als Gefäßersatz in die Halsschlagader einer Ratte implantiert. Dabei hat sich mikrokristalline Cellulose als formstabiles biokompatibles Material erwiesen.

Es ist aus dem Stand der Technik bekannt, Cellulose bildende Bakterien in einer Standkultur an der Grenzschicht zwischen einem wässrigen Nährmedium und Luft zu kultivieren. Dabei bildet sich eine auf dem Nährmedium schwimmende Celluloseschicht (sog. "Pellikel"). Es wird vorgeschlagen, dieses Pellikel als Wundauflage einzusetzen. Bei der Herstellung von Cellulosekörpern nach dieser Methode hat sich gezeigt, dass sich die Reaktionsbedingungen in der Standkultur ändern, weil Nährstoffe im Nährmedium verbraucht werden und sich Stoffwechselprodukte der Cellulose bildenden Bakterien im Nährmedium anreichern. Dadurch kann sich der pH-Wert des Nährmediums ändern. Außerdem können sich die veränderten Reaktionsbedingungen auf die Qualität der gebildeten Cellulose auswirken, so dass Celluloseschichten mit veränderter, inhomogener Struktur und Dichte entstehen können. Insbesondere kann eine zunehmende Verfestigung in Richtung der der Luft zugewandten Seite der Celluloseschicht beobachtet werden. Die Strukturveränderungen können so stark ausgeprägt sein, dass die Ausbildung einzelner Schichten (Delaminierung), die sich unter der Einwirkung von Scherkräften voneinander lösen können, beobachtet werden kann.

Die EP 0 396 344 A1 offenbart ein Verfahren zur Herstellung eines Cellulose enthaltenden Körpers unter Verwendung Cellulose produzierender Mikroorganismen, die von einer Nährlösung versorgt werden. Dabei wird ebenfalls eine Membran verwendet, die sauerstoffdurchlässig und für die Nährlösung und die Mikroorganismen undurchlässig ist. Der Sauerstoff wird dabei auf einer ersten Seite der Membran bereitgestellt, so dass dieser durch die Membran hindurch auf die zweite Seite gelangen kann.

Die WO 02/081725 A1 offenbart ebenfalls ein Verfahren zur Herstellung eines Cellulose enthaltenden Körpers unter Verwendung Cellulose produzierender Mikroorganismen, die von einer Nährlösung versorgt werden. Die Nährstofflösung wird dabei mittels eines Erosolerzeugers auf ein Cellulosesubstrat aufgesprüht.

Aus der WO 2008/040729 A2 ist ferner ein Verfahren zur Herstellung von hohlen Cellulosekörpern unter Verwendung Cellulose produzierender Mikroorganismen bekannt, die von einer Nährlösung versorgt werden. Die Mikroorganismen werden dabei auf der Außenfläche einer Hohlform gezüchtet; an der Innenfläche wird ein Sauerstoff enthaltendes Gas bereitgestellt.

### Der Erfindung zugrunde liegendes Problem

Der Erfindung liegt daher die Aufgabe zugrunde, ein weiteres Verfahren und eine Vorrichtung zur Herstellung Cellulose enthaltender Körper unter Verwendung Cellulose produzierender Mikroorganismen, die von einer Nährlösung versorgt werden, bereitzustellen.

### Erfindungsgemäße Lösung

Zur Lösung der Aufgabe lehrt die Erfindung ein Verfahren zur Herstellung eines Cellulose enthaltenden Körpers, bei dem Cellulose bildende Mikroorganismen auf einer Seite einer Membran, auf der eine sauerstoffhaltige Atmosphäre herrscht, angeordnet werden und dort Cellulose bilden. Die Mikroorganismen werden von einer Nährlösung versorgt, die im Wesentlichen auf der anderen Seite der Membran vorgesehen ist. Die Membran ist dabei für die Mikroorganismen und die Cellulose im Wesentlichen undurchlässig, für die Nährlösung jedoch durchlässig. Wegen dieser Eigenschaft der Membran kann die Nährlösung durch die Membran auf die Seite der Mikroorganismen hindurch treten und dort die Mikroorganismen versorgen.

Die Erfindung lehrt außerdem eine Anordnung zur Herstellung eines oder mehrerer Cellulosekörper, umfassend ein Gefäß mit einer Membran, die für die Mikroorganismen und die Cellulose im Wesentlichen undurchlässig, für die Nährlösung jedoch durchlässig ist. Gemäß der Erfindung befindet sich auf einer ersten Seite der Membran eine Kammer für die Nährlösung, und auf der anderen Seite der Membran eine zweite Kammer mit einer Gasathmosphäre, in der auch die Cellulose erzeugenden Mikroorganismen vorgesehen sind.

Mit Hilfe des erfindungsgemäßen Verfahrens und der Vorrichtung lassen sich Cellulosekörper herstellen, die eine besonders homogene Struktur und Dichte aufweisen.

Während des Herstellungsprozesses werden das Nährmedium und/oder die Gasathmoshpäre vorzugsweise ausgetauscht bzw. aufgefrischt, um möglichst gleichmäßige Wachstums- bzw. Reaktionsbedingungen für die Mikroorganismen bereit zu stellen. Von besonderer Relevanz sind dabei insbesondere die Konzentration von Nährstoffen und der pH-Wert am Ort der Cellulose bildenden Mikroorganismen.

Der Austausch von Nährmedium und/oder Gasathmosphäre kann geregelt werden. In diesem Fall ist eine Regelung mit einer Sensorik, wie z.B. einem pH-Sensor, und einem Stellglied, wie z. B. einem steuerbaren Ventil, das den Zu- oder Abfluss eines Mediums regelt, vorzusehen. Neben dem pH-Wert können selbstverständlich auch andere physikalische Größen geregelt werden.

In der Nährlösung befinden sich vorzugsweise keine Cellulose bildende Mikroorganismen. Wegen der semipermeablen Eigenschaft der Membran gelangen auch während des Herstellungsprozesses keine Cellulose bildenden Mikroorganismen in die Nährlösung. Dadurch kann insbesondere eine Pelletbildung in der Nährlösung und die Bildung von Cellulose-Ablagerungen in zu- oder abführenden Leitungen verhindert werden.

Die Erfindung kann z. B. dazu eingesetzt werden, Cellulose umfassende Medizinprodukte mit einer vorgegebenen dreidimensionalen Form herzustellen. Insbesondere lassen sich mit der Erfindung Wundauflagen sowie strukturierte Implantate, z.B. Gefäßprothesen, herstellen.

Mit der Erfindung können auch Cellulosekörper zur Verwendung außerhalb der Medizin hergestellt werden, z.B. für Lautsprechermembranen oder elektronisches Papier. Für alle genannten Anwendungen kann insbesondere die hohe mit dem erfindungsgemäßen Verfahren erreichbare Homogenität des Cellulosekörpers von Vorteil sein.

Eine bevorzugte Membran besteht aus einem hydrophilen Material, vorzugsweise einem Polymer oder einem anorganischen, nichtmetallischen Werkstoff, der einen Thermoprozess durchlaufen hat, vorzugsweise Keramik. Bevorzugt sind Membranmaterialien, die Poren aufweisen, z.B. Mikro- oder Ultrafiltrationsmembranen. Mit dieser Ausführung der Erfindung ist erreichbar, dass die Poren sich durch Kontakt zu dem Vorrat mit dem Nährmedium befüllen. Mit dieser Ausführung der Erfindung ist erreichbar, dass die Kapillarkräfte ausreichen, um Nährmedium durch die Membran zu transportieren. Die Membran ist vorzugsweise mit Dampf sterilisierbar.

In einer speziellen Ausführung der Erfindung sind nur Teile der Membran für die Nährlösung durchlässig ausgebildet. Dadurch sind ein oder mehrere Bereiche der Membran bestimmbar, in denen sich ein oder mehrere Cellulosekörper ausbilden.

Die Membran kann mit einer Stützschicht, z.B. einem Gitter, ausgestattet werden, um die Membran gegen die Last des oder der Cellulosekörper abzustützen.

Gemäß einer ersten Ausführungsform der Erfindung ist die Membran als eine ebene, im Wesentlichen horizontal verlaufende Fläche gebildet. Die Nährlösung befindet sich in diesem Fall im Wesentlichen unterhalb der Membran in einem Gefäß, und die Cellulose bildenden Mikroorganismen oberhalb der Membran. Wegen der semipermeablen Eigenschaft der Membran kann das Nährmedium durch die Membran hindurch nach oben dringen und die Mikroorganismen versorgen. Die Mikroorganismen können jedoch nicht in die Nährlösung übergehen. Die Membran ist vorzugsweise geringfügig in die Nährlösung eingetaucht, so dass sich auf der Membran eine dünne, z. B. wenige mm dicke Schicht aus Nährlösung bildet. Im Grunde ist es bereits ausreichend, wenn die Nährlösung die Oberfläche der Membran benetzt.

Gemäß einer zweiten Ausführungsform der Erfindung bildet die Membran eine Umfangsfläche einer Hohlform. Diese Hohlform kann z. B. ein lang gestreckter Körper, wie z. B. ein Hohlzylinder, vorzugsweise ein Kreiszylinder, sein. Bei dieser Ausführungsform befinden sich die Cellulose bildenden Mikroorganismen vorzugsweise im Innenraum der Hohlform, während die Nährlösung außerhalb der Hohlform vorgesehen ist. Die umgekehrte Anordnung ist aber gleichfalls möglich. Die Nährlösung kann wiederum durch die Membran hindurch diffundieren und die im Innenraum befindlichen Mikroorganismen mit Nährstoffen versorgen. Der Innenraum der Hohlform ist vorzugsweise nur teilweise mit einer Suspension aus Cellulose bildenden Mikroorganismen gefüllt. In einem anderen Teil des Innenraums herrscht Gasathmosphäre.

Die Hohlform kann einen inneren Formkern, wie z. B. einen zentral angeordneten Stab aufweisen. Der Formkern kann irreversibel verformbar sein, wie es z.B. in den deutschen Patentanmeldungen DE 10 2007 006 843 und DE 10 2007 006 844 beschrieben ist. Der Cellulose-Hohlkörper kann zumindest teilweise nach dem in der deutschen Patentanmeldung DE 10 2007 016 852 offenbarten Verfahren hergestellt sein. Die Membran kann auch in die Wandung einer Hohlform integriert werden, indem sie z. B. die Unterlage bildet, auf die die Form nach dem Verfahren der DE 10 2007 016 852 schichtweise aufgebaut wird. Es können auch mehrere Innenräume vorgesehen sein. Dies kann vorteilhaft die Herstellung größerer Stückzahlen erleichtern. Vorzugsweise sind alle Innenräume im Wesentlichen gleich ausgebildet.

Im Innenraum der Hohlform befindet sich vorzugsweise auch ein Gas, insbesondere ein sauerstoffhaltiges Gas, wie z. B. Luft, um die Mikroorganismen mit dem für den Wachstumsprozess erforderlichen Gas zu versorgen.

Gemäß einer bevorzugten Ausführung der Erfindung wird der Durchtritt des Nährmediums durch die Membran geregelt. Dies kann z. B. über eine Variation des Gasdrucks, insbesondere des Wasserdampfpartialdrucks im Innenraum der Hohlform und/oder des hydrostatischen Drucks der Nährlösung erfolgen. Dadurch kann eine im Wesentlichen gleichmäßige Transportrate von Nährstoffen zu den Cellulose bildenden Mikroorganismen erreicht werden. Der hydrostatische Druck an der Membran kann z.B. dadurch eingestellt werden, dass die Hohlform unterschiedlich tief in den Vorrat des Nährmediums eingetaucht wird.

Gemäß einer speziellen Ausführungsform der Erfindung ist die lang gestreckte Hohlform so weit in die Nährflüssigkeit eingetaucht, dass im Wesentlichen die gesamte Außenseite der Membran mit der Nährlösung in Kontakt ist. Vorzugsweise ist die gesamte Hohlform in den Nährflüssigkeitsvorrat eingetaucht. Im Inneren der Hohlform herrscht vorzugsweise ein Gasüberdruck, um zu erreichen, dass sie nicht mit Nährflüssigkeit vollläuft, sondern nur so viel Nährflüssigkeit durch die Membran dringt, wie für die Versorgung der Cellulosebakterien erwünscht ist.

Gemäß einer anderen Ausführung der Erfindung ist die lang gestreckte Hohlform so weit in den Nährflüssigkeitsvorrat eingetaucht, dass zu jedem Zeitpunkt nur ein Teil der Membran mit Nährflüssigkeit in Kontakt ist. Im Inneren der Hohlform herrscht vorzugsweise Normaldruck.

Die Hohlform erstreckt sich vorzugsweise in horizontaler Richtung. Die Wachstumsrichtung der Cellulose verläuft damit im Wesentlichen quer zur Längsrichtung der Hohlform.

Die Hohlform ist vorzugsweise um ihre Längsachse rotierbar angeordnet, um hydrostatische Druckunterschiede und den ungleichen Einfluss der Schwerkraft auf das Cellulosewachstum im zeitlichen Mittel auszugleichen. Die Hohlform wird in diesem Fall während des Herstellungsprozesses langsam drehend angetrieben.

In einer bevorzugten Ausführung der Erfindung ist im Innenraum der Hohlform eine weitere Membran vorgesehen, die für die Mikroorganismen und die Cellulose im Wesentlichen undurchlässig, für ein Gas, insbesondere Sauerstoff, jedoch durchlässig ist. Bevorzugte Materialien für die zweite Membran sind PTFE, FEP, PFA, TFA, PFP, TTE, TPE-E, MVQ, MFQ, PEEK, PET, TBT, PVC, PMMA, PPS, Celluloseester, Polyamid, Gummi, insbesondere Latex oder Naturkautschuk, Polypropylen, Polycarbonat, Polystyrol, Polyurethan oder Silikon. Die zweite, innere Membran definiert zusammen mit der ersten, äußeren Membran einen Hohlraum, in dem die Cellulose wächst. Die Kontur der Membran bestimmt dabei die spätere Form der hergestellten Cellulosekörper. Wenn die zweite Membran konzentrisch zur ersten Membran angeordnet ist, wird beispielsweise ein ringförmiger Hohlraum definiert. Auf diese Weise kann z. B. eine Blutgefäß-Prothese aus Cellulose hergestellt werden. Der Hohlraum ist vorzugsweise abgeschlossen, um das Eindringen von Verunreinigungen zu verhindern. Dies ist insbesondere bei der Herstellung von Cellulosekörpern für medizinische Zwecke vorteilhaft.

Die zweite Membran ist vorzugsweise mit Dampf sterilisierbar. Sie kann Bestandteil eines festen Körpers sein, um somit eine höhere Stabilität zu erreichen.

Eine solche zweite Membran bzw. ein Membrankörper kann grundsätzlich in jeder erfindungsgemäßen Vorrichtung verwendet werden, um den Cellulosekörpern eine gewünschte Form zu geben. So kann z. B. auch auf der horizontal angeordneten Membran eine zweite Membran angeordnet sein, die zusammen mit der ersten Membran einen oder mehrere Hohlräume bildet, in denen die Cellulosekörper wachsen.

Die beiden Membranen sind vorzugsweise an wenigstens einer Stelle miteinander verklebt, verschweißt oder anderweitig verbunden. Der zwischen den Membranen gebildete Hohlraum kann mehrere Unterteilungen aufweisen. Vorzugsweise ist jeder Innenraum mit Cellulose bildenden Mikroorganismen beimpft. Durch diese Ausführung der Erfindung lassen sich eine große Zahl von Cellulosekörpern gleichzeitig herstellen.

Die Herstellung der Cellulosekörper erfolgt vorzugsweise in einem Gefäß, vorzugsweise einem wannenartigen Gefäß. Das Gefäß ist - abgesehen von optionalen Öffnungen für den Austausch von Nährmedium und/oder Gas - vorzugsweise geschlossen. Es umfasst vorzugsweise ein Bodenteil und einen Deckel.

Nachdem der Cellulosekörper die gewünschte Höhe erreicht bzw. die Hohlform vollständig ausgefüllt hat, kann er von der Membran gelöst werden. Die erzeugten Cellulosekörper werden anschließend in bekannter Weise gereinigt, z.B. wie in DE 10 2006 007 412 offenbart. Die Cellulose ist vorzugsweise mikrobielle Cellulose, besonders vorzugsweise mikrokristalline Cellulose. Die Cellulose bildenden Mikroorganismen sind vorzugsweise Bakterien, besonders vorzugsweise Bakterien des Stamms *Gluconacetobacter xylinus.* Es ist denkbar, dass auch noch andere Cellulose bildende Mikroorganismen eingesetzt werden, z.B. geeignete Stämme vom Agrobacterium, Rhizobium, Sarcina, Pseudomonas, Achromobacter, Aerobacter und Cooglea. Da die Gene der Cellulose synthetisierenden Enzymkomplexe von *Gluconacetobacter xylinus* bekannt sind, können diese auch in andere Mikroorganismen wie z.B. *Escherichia coli* durch die Anwendung bekannter molekularbiologischer Verfahren gebracht werden, wodurch auch diese Mikroorganismen als Cellulose bildende Mikroorganismen in Frage kommen.

Für die Kultivierung Cellulose bildender Bakterien sind unterschiedliche Nährmedien beschrieben. Ein geeignetes, häufig verwendetes Medium ist das im Biochemical Journal 58 von 1954, Seiten 345-352 beschriebene Medium von Schramm und Hestrin. Ein Nachteil dieses Mediums kann darin bestehen, dass es nicht genau definiert ist, da es Hefeextrakt und Pepton enthält.

Für die Ausführung der vorliegenden Erfindung wird ein vollsynthetisches Medium bevorzugt, wie z.B. von Forng et al. in Applied and Environmental Microbiology von 1989, Band 55, Nr. 5, Seiten 1317-1319 beschrieben. Ein Nachteil dieses Mediums kann in dem etwas langsameren Wachstum der Bakterien bestehen.

Es ist auch denkbar, den sogenannten Kombucha-Teepilz zur Ausführung der Erfindung zu verwenden. Diese Kultur enthält neben *Gluconacetobaeter xylinus* zahlreiche andere in Symbiose lebende Mikroorganismen, wie Hefen und Bakterien, und lässt sich durch ein Medium, bestehend lediglich aus Schwarztee und Saccharose, (100 g/1) unterhalten.

### Kurzbeschreibung der Zeichnungen

Die Erfindung wird im folgenden anhand schematischer Zeichnungen und Ausführungsbeispielen mit weiteren Einzelheiten näher erläutert. Es zeigen:
Fig. 1: eine schematische Darstellung des Arbeitsprinzips einer ersten Ausführung der Erfindung;
Fig. 2: eine perspektivische Darstellung eines Ausführungsbeispiels der Erfindung, das nach den in Fig. 1 dargestellten Prinzip arbeitet;

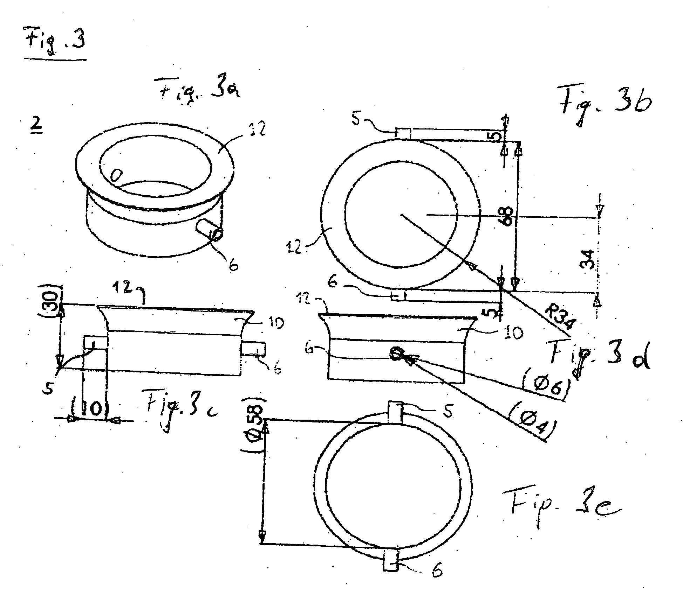

Fig. 3: eine Darstellung einer der beiden identischen Kammern des Ausführungsbeispiels nach Fig. 2, (a) in perspektivischer Ansicht, (b) von oben, (c) in einer ersten Seitenansicht, bei der die Zu- und Abführungen parallel zur Bildebene verlaufen, (d) in einer dazu um 90° gedrehten Seitenansicht und (e) in Querschnittsansicht durch die Kammer auf Höhe der Zu- und Abführungen;
Fig. 4: ein Ausführungsbeispiel der Erfindung, bei dem die Membran einen röhrenförmigen Innenraum bildet, in Querschnittsansicht senkrecht zur Längsachse der Röhre;
Fig. 5: ein weiteres Ausführungsbeispiel mit einem röhrenförmigen Innenraum in Querschnittsansicht senkrecht zur Längsachse der Röhre;
Fig. 6: ein Ausführungsbeispiel der Erfindung mit einer ersten und einer zweiten Membran, die nach Art einer Blisterverpackung verbunden sind.

### Beschreibung anhand von Ausführungsbeispielen

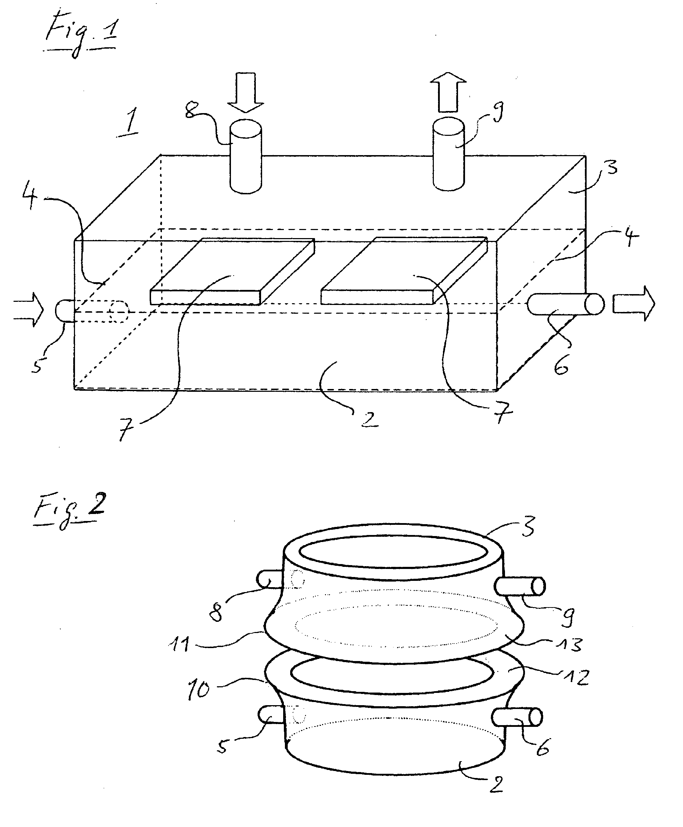

Das Funktionsprinzip eines ersten Ausführungsbeispiels der Erfindung ist in Fig. 1 erläutert. Ein Gefäß 1 umfasst eine erste Kammer 2 für Nährmedium und eine zweite Kammer 3, in der die Cellulose bildenden Mikroorganismen, z.B. Bakterien vom Stamm *Gluconacetobacter xylinus* vorliegen. Die beiden Kammern sind durch eine Membran 4 aus hydrophilem PES getrennt. Die erste Kammer 2 ist vollständig mit Nährmedium gefüllt, wobei ständig durch die Zuführung 5 neues Nährmedium zufließt und durch die Abführung 6 Nährmedium abfließt. Auf diese Weise können die das Nährmedium betreffenden Reaktionsbedingungen, d.h. insbesondere die Zusammensetzung und der pH-Wert des Kulturmediums kontrolliert werden. An den Stellen, an denen die Membran 4 für das Nährmedium durchlässig ist, durchdringt das Nährmedium, vorzugsweise durch den Kapillareffekt zumindest mit angetrieben, die Membran 4 und gelangt so in die zweite Kammer 3, wo es durch den sich aufbauenden Cellulosekörper 7 mittels Diffusion an dessen Oberfläche gelangt und dort die Cellulose bildenden Bakterien versorgt. Die Nährlösung besteht z.B. aus 20 g Glucose, 5 g Hefeextrakt, 5 g Bactopepton, 2,7 g Natriumphosphat und 1,15 g Citronensäure-Monohydrat, 0,5 g Magnesiumsulfatheptahydrat in einem Liter Wasser und hat einen pH-Wert von etwa 6,0. Außerdem wird die zweite Kammer 3 über die Gaszuführung 8 und den Gasabführung 9 mit einem sauerstoffhaltigen Gasgemisch zur Vorsorgung der Cellulose bildenden Mikroorganismen durchströmt.

Eine Vorrichtung, die nach dem beschriebenen Prinzip arbeitet, ist das in Fig. 2 dargestellte Gefäß 1. Die erste Kammer 2 und die zweite Kammer 3 umfassen identischen, halbgeschlossenen und mit Flanschen 10, 11 versehenen Zylindern aus Glas, wobei die Flansche 10, 11 mit Schliffflächen 12, 13 versehen sind. Zwischen den Schliffflächen 12, 13 wird die Membran (nicht dargestellt) mit Silikonflachdichtung (nicht dargestellt) eingespannt. Über die jeweils im Winkel von 180° zueinander angeordneten Zu- und Abführungen 5, 6 wird die erste Kammer 2 im Betrieb kontinuierlich so mit Nährmedium durchströmt, dass die kreisförmige, dem Innenraum der ersten Kammer 2 zugewandte Teil der Membran 4 vollständig von dem Nährmedium benetzt wird. Die zweite Kammer 3 wird während des Betriebs durch die Zu- 8 und die Abführungen 9 mit steriler Luft durchströmt, deren Wasserdampfpartialdruck auf einen Wert von 0,2 bar eingestellt wird.

Vor dem Betrieb wird das Gefäß 1 mit eingespannter Membran dampfsterilisiert und dann die der zweiten Kammer zugewandte Seite der Membran 4 unter sauberen und keimfreien Bedingungen, vorzugsweise an einer Sterilbank, mit einer Suspension des *Cluconacetobacter xylinus* aus einer Vorkultur, z.B. eine 3 Tage alten Vorkultur aus *Gluconacetobacter xylinus* (DSMZ Braunschweig), so überspült, dass eine im Wesentlichen gleichmäßige Belegung der Membranoberfläche mit den Bakterien erreicht wird. Dabei läuft das Medium über die hydrophile Membran 4 in die erste Kammer 2 des Gefäßes 1, die Bakterien werden jedoch von der Membran 4 in der zweiten Kammer 3 zurückgehalten. Dann wird das Gefäß 1 unter der Sterilbank geschlossen, in einem Inkubator untergebracht, um die Reaktionstemperatur zu kontrollieren, und dann mit Sterilkupplungen an den Zu- und Abführungen mit Versorgungsleitungen für die Luft und das Nährmedium verbunden. Dabei wird die erste Kammer 2 im Bypass zu einem Rührkesselreaktor betrieben, mit dem sich die Reaktionsbedingungen im Nährmedium, insbesondere der pH, einfach kontrollieren lassen. Der Aufbau des Cellulosekörpers 7 in der zweiten Kammer 3 wird durch den transparenten Glaszylinder mit dem Auge beobachtet. Nach 10 Tagen wird das Gefäß 1 geöffnet, der gebildete Cellulosekörper 7 von der Oberfläche der Membran 4 abgezogen und anschließend in kochendem Wasser gereinigt und danach sterilisiert.

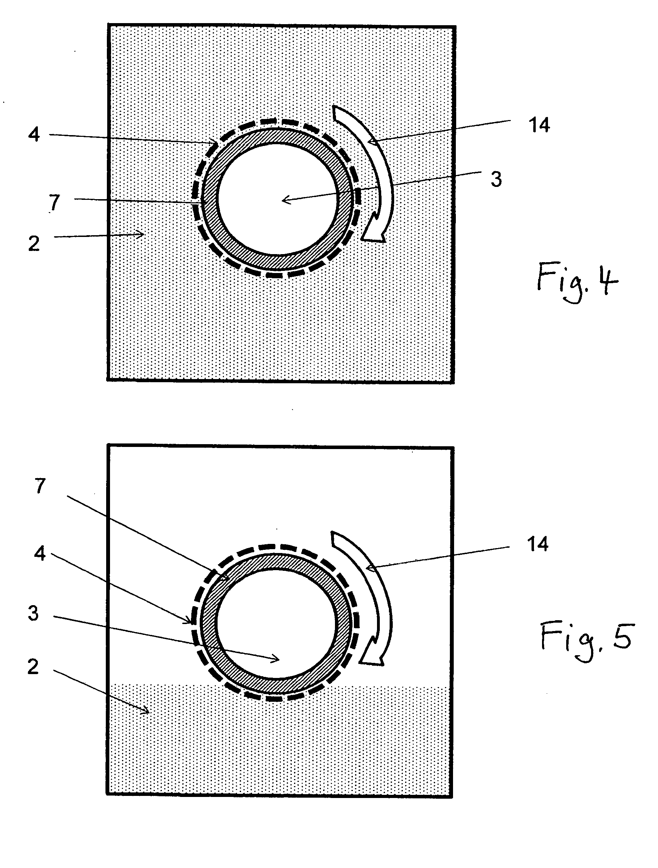

Ein weiteres Ausführungsbeispiel der Erfindung ist in Fig. 4 dargestellt. Dabei ist die Membran 4 zu einem langgestreckten Kreiszylinder geschlossen, der in Fig. 4 im Querschnitt dargestellt ist. Der Kreiszylinder ist in einen Vorrat von Nährmedium vollständig eingetaucht. Das Innere des Kreiszylinders ist mit den Cellulose bildenden Mikroorganismen beimpft und durch die Membran 4 diffundiert das Nährmedium in das Innere des Zylinders, so dass sich auf der Innenseite des Zylinders ein ebenfalls kreiszylinderförmiger Cellulosehohlkörper 7 bildet. Zur Versorgung der Mikroorganismen mit Sauerstoff wird der Kreiszylinder mit Luft durchströmt. Dabei herrscht, um zu verhindern, dass der Kreiszylinder mit Nährmedium voll läuft, im Kreiszylinder ein Überdruck. Außerdem wird der Kreiszylinder in Richtung des Pfeils 14 langsam gedreht, um hydrostatische Druckunterschiede und den ungleichen Einfluss der Schwerkraft auf das Cellulosewachstum auszugleichen.

Eine Variante der zuletzt beschriebenen Ausführungsform ist in Fig. 5 zu sehen. Hier ist der Zylinder nicht vollständig in den Vorrat 2 des Kulturmediums eingetaucht, sondern nur zum Teil. Dadurch ist es nicht mehr erforderlich, für einen Überdruck im Inneren des Zylinders zu sorgen. Auch hier wird der Zylinder langsam gedreht, so dass alle Bereiche der Membran 4 in Kontakt mit dem Nährmedium kommen und hydrostatische Druckunterschiede sowie der ungleiche Einfluss der Schwerkraft auf das Cellulosewachstum ausgeglichen werden können.

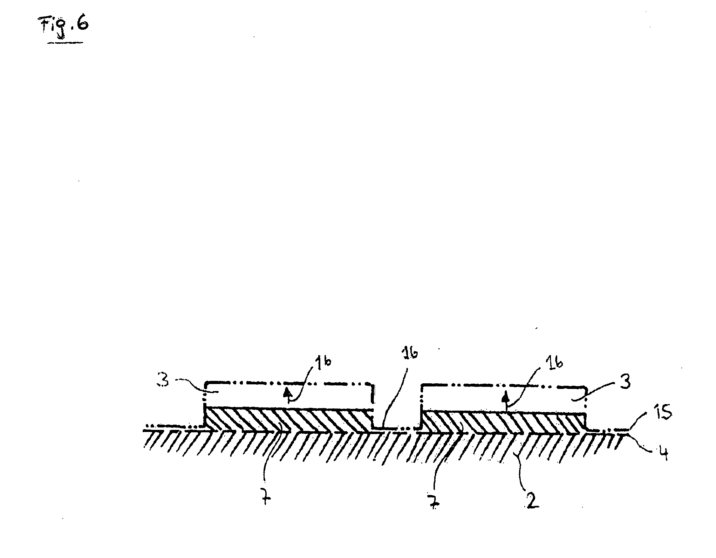

Ein weiteres Ausführungsbeispiel der Erfindung ist in Fig. 6 wiedergegeben. Hier sind mehrere zweite Kammern 3 vorgesehen, die jeweils von der Membran 4 und einer zweiten Membran 15 gebildet werden. Zu diesem Zweck ist die zweite Membran 15, in anderen möglichen Ausführungen auch die erste Membran 4, oder beide Membrane, mit mehreren die zweiten Kammern 3 bildenden Auswölbungen ausgestattet und die erste Membran 4 und die zweite Membran 15 sind in den Bereichen 16 zwischen diesen Auswölbungen miteinander verklebt. Die zweiten Kammern 3 bilden Hohlformen, die die Cellulose 7 hineinwächst, bis die zweiten Kammern 3 vollständig mit Cellulose 7 ausgefüllt sind. Nährmedium dringt aus einem Vorrat 2 von Nährmedium durch die Membran 4 in die zweite Kammern 3 hinein, wo es durch den Cellulosekörper 7 an dessen Oberfläche diffundiert, an der sich die Cellulose bildenden Mikroorganismen befinden. Die Membran 15 ist gasdurchlässig, so dass die Mikroorganismen durch die Membran 15 von außen mit dem erforderlichen Sauerstoff versorgt werden können. Die Wachstumsrichtung der Cellulosekörper ist mit 16 bezeichnet.

Die in der vorstehenden Beschreibung, den Ansprüchen und den Zeichnungen offenbarten Merkmale können sowohl einzeln als auch in beliebiger Kombination für die Verwirklichung der Erfindung in ihren verschiedenen Ausgestaltungen von Bedeutung sein.

## Patentansprüche

1. Verfahren zur Herstellung eines Cellulose enthaltenden Körpers (7) mit Hilfe von Cellulose erzeugenden Mikroorganismen, die von einer Nährlösung versorgt werden, **gekennzeichnet durch**
- das Bereitstellen einer Membran (4), die für die Nährlösung durchlässig und für die Mikroorganismen im Wesentlichen undurchlässig ist,
- das Bereitstellen von Nährlösung auf einer ersten Seite der Membran (4), so dass sie durch die Membran (4) hindurch auf die andere, zweite Seite treten kann,
- das Bereitstellen einer Gasathmosphäre auf der zweiten Seite der Membran (4), und
- das Bereitstellen von Cellulose erzeugenden Mikroorganismen auf der zweiten Seite der Membran (4), so dass die Mikroorganismen von der **durch** die Membran (4) hindurch tretenden Nährlösung versorgt werden.

2. Verfahren nach Anspruch 1,
**dadurch gekennzeichnet, dass**
die Membran (4) eine Keramikmembran ist.

3. Verfahren nach Anspruch 1 oder 2,
**dadurch gekennzeichnet, dass**
die Membran (4) als ebene Schicht angeordnet wird, wobei die Nährlösung im Wesentlichen unterhalb der Membran (4), und die Cellulose erzeugenden Mikroorganismen oberhalb der Membran (4) angeordnet sind.

4. Verfahren nach Anspruch 1 oder 2,
**dadurch gekennzeichnet, dass**
die Membran (4) einen geschlossenen Umfang einer Hohlform bildet.

5. Verfahren nach Anspruch 4,
**dadurch gekennzeichnet, dass**
die Cellulose erzeugenden Mikroorganismen im Innenraum der Hohlform angeordnet sind.

6. Verfahren nach einem der vorherigen Ansprüche,
**dadurch gekennzeichnet, dass**
während des Herstellungsprozesses ein Austausch der Nährlösung stattfindet.

7. Verfahren nach einem der vorherigen Ansprüche,
**dadurch gekennzeichnet, dass**
während des Herstellungsprozesses ein Austausch der Gasatmosphäre stattfindet.

8. Verfahren nach einem der vorherigen Ansprüche,
**dadurch gekennzeichnet, dass**
der Durchtritt von Nährlösung durch die Membran (4) mittels des Gasdrucks oder eines partialen Gasdrucks geregelt wird.

9. Verfahren nach einem der vorherigen Ansprüche,
**dadurch gekennzeichnet, dass**
der Durchtritt von Nährlösung durch die Membran (4) mittels des hydrostatischen Drucks der Nährlösung an der Membran (4) geregelt wird.

10. Verfahren nach einem der vorherigen Ansprüche,
**dadurch gekennzeichnet, dass**
auf der Seite der Cellulose erzeugenden Mikroorganismen eine zweite Membran (15) vorgesehen wird, die für die Mikroorganismen und die Cellulose im Wesentlichen undurchlässig, für ein Gas oder einen Bestandteil des Gases jedoch durchlässig ist.

11. Verfahren nach Anspruch 10,
**dadurch gekennzeichnet, dass**
die zweite Membran (15) zusammen mit der ersten Membran (4) einen Hohlraum definiert, in dem der Cellulosekörper (7) wächst.

12. Anordnung zur Herstellung eines Cellulose enthaltenden Körpers mit Hilfe von Cellulose bildenden Mikroorganismen, die von einer Nährlösung versorgt werden, mit einem Gefäß (1) mit einer Membran (4), die für die Mikroorganismen und die Cellulose im Wesentlichen undurchlässig, für die Nährlösung jedoch durchlässig ist
**dadurch gekennzeichnet, dass**
das Gefäß auf einer ersten Seite der Membran (4) eine Kammer (2) für die Nährlösung, und auf der anderen Seite der Membran (4) eine zweite Kammer (3) mit einer Gasathmosphäre aufweist, in der auch die Cellulose erzeugenden Mikroorganismen vorgesehen sind.

13. Anordnung nach Anspruch 12,
**dadurch gekennzeichnet, dass**
das Gefäß (1) wenigstens eine Öffnung (5,6;8,9) für einen Austausch von Gas und/oder Nährlösung aufweist.

14. Anordnung nach Anspruch 12 oder 13,
**dadurch gekennzeichnet, dass**
das Gefäß (1), abgesehen von wenigstens einer optional vorhandenen Öffnung (5,6;8,9) für einen Austausch von Gas und/oder Nährlösung geschlossen ist.

15. Anordnung nach einem der Ansprüche 12 bis 14,
**dadurch gekennzeichnet, dass**
eine zweite Membran (15) vorgesehen ist, die in Verbindung mit der ersten Membran (4) einen oder mehrere Hohlräume definiert, in dem bzw. denen der bzw. die Cellulosekörper (7) wachsen.

## Claims

1. Method to produce a body (7) containing cellulose with the help of microorganisms that create cellulose, which are fed by a nutrient solution,
**characterised by**
- the provision of a membrane (4), which is permeable for the nutrient solution and substantially impermeable for the microorganisms,
- the provision of a nutrient solution on a first side of the membrane (4), such that it can pass through the membrane (4) to the other, second side,
- the provision of a gaseous atmosphere on the second side of the membrane (4), and
- the provision of microorganisms that create cellulose on the second side of the membrane (4), such that the microorganisms are fed by the nutrient solution passing through the membrane (4).

2. Method according to claim 1,
**characterised in that**,
the membrane (4) is a ceramic membrane.

3. Method according to claim 1 or 2,
**characterised in that**,
the membrane (4) is arranged as an even layer, wherein the nutrient solution is arranged substantially underneath the membrane (4) and the microorganisms that create cellulose are arranged above the membrane (4).

4. Method according to claim 1 or 2,
**characterised in that**,
the membrane (4) forms a closed periphery of a hollow shape.

5. Method according to claim 4,
**characterised in that**,
the microorganisms that create cellulose are arranged in the inner space of the hollow shape.

6. Method according to one of the preceding claims,
**characterised in that**,
during the production process, an exchange of nutrient solution takes place.

7. Method according to one of the preceding claims,
**characterised in that**,
during the production process, an exchange of the gaseous atmosphere takes place.

8. Method according to one of the preceding claims,
**characterised in that**,
the passing of nutrient solution through the membrane (4) is regulated by means of the gas pressure or a partial gas pressure.

9. Method according to one of the preceding claims,
**characterised in that**,
the passing of nutrient solution through the membrane (4) is regulated by means of the hydrostatic pressure of the nutrient solution at the membrane (4).

10. Method according to one of the preceding claims,
**characterised in that**,
a second membrane (15) is provided on the side of the microorganisms that create cellulose, which is substantially impermeable for the microorganisms and the cellulose, but permeable for a gas or a component of the gas.

11. Method according to claim 10,
**characterised in that**,
the second membrane (15) defines a hollow space together with the first membrane (4), in which the cellulose body (7) grows.

12. Arrangement for the production of a body containing cellulose with the help of microorganisms that form cellulose, which are fed by a nutrient solution, having a container (1) with a membrane (4) which is substantially impermeable for the microorganisms and the cellulose, but is permeable for the nutrient solution
**characterised in that**,
the container has a chamber (2) for the nutrient solution on a first side of the membrane (4) and a second chamber (3) having a gaseous atmosphere on the other side of the membrane (4), in which the microorganisms that create cellulose are also provided.

13. Arrangement according to claim 12,
**characterised in that**,
the container (1) has at least one opening (5, 6; 8, 9) for an exchange of gas and/or nutrient solution.

14. Arrangement according to claim 12 or 13,
**characterised in that**
the container (1) is closed except for at least one optionally present opening (5, 6; 8, 9) for an exchange of gas and/or nutrient solution.

15. Arrangement according to one of claims 12 to 14,
**characterised in that**,
a second membrane (15) is provided, which defines one or several hollow spaces in connection with the first membrane (4), in which second membrane(s) (15) the cellulose bodies (7) grow.

## Revendications

1. Procédé de fabrication d'un corps (7) contenant de la cellulose utilisant des microorganismes produisant de la cellulose, lesquels sont alimentés par une solution nutritive,
**caractérisé par**
- la mise à disposition d'une membrane (4), principalement perméable à la solution nutritive et imperméable aux microorganismes,
- la mise à disposition de solution nutritive d'un premier côté de la membrane (4), de sorte qu'elle peut traverser la membrane et passer de l'autre côté,
- la mise à disposition d'une atmosphère gazeuse du second côté de la membrane (4), et
- la mise à disposition de microorganismes produisant de la cellulose du second côté de la membrane (4), de sorte que les microorganismes sont alimentés par la solution nutritive qui passe à travers ladite membrane (4).

2. Procédé selon la revendication 1,
**caractérisé en ce que** la membrane (4) est une membrane en céramique.

3. Procédé selon la revendication 1 ou 2,
**caractérisé en ce que** la membrane (4) forme une couche plane, la solution nutritive étant principalement disposée en-dessous de la membrane (4) et les microorganismes produisant la cellulose étant disposés au-dessus de la membrane (4).

4. Procédé selon la revendication 1 ou 2,
**caractérisé en ce que** la membrane (4) forme une enveloppe fermée de forme creuse.

5. Procédé selon la revendication 4,
**caractérisé en ce que** les microorganismes produisant de la cellulose sont disposés à l'intérieur de la forme creuse.

6. Procédé selon l'une quelconque des revendications précédentes,
**caractérisé en ce qu'**un échange de solution nutritive a lieu pendant le processus de fabrication.

7. Procédé selon l'une quelconque des revendications précédentes,
**caractérisé en ce qu'**un échange d'atmosphère gazeuse a lieu pendant le processus de fabrication.

8. Procédé selon l'une quelconque des revendications précédentes,
**caractérisé en ce que** le passage de la solution nutritive à travers la membrane (4) est réglé au moyen de la pression gazeuse ou d'une pression gazeuse partielle.

9. Procédé selon l'une quelconque des revendications précédentes,
**caractérisé en ce que** le passage de la solution nutritive à travers la membrane (4) est réglé au moyen de la pression hydrostatique de la solution nutritive au niveau de la membrane (4).

10. Procédé selon l'une quelconque des revendications précédentes,
**caractérisé en ce qu'**une seconde membrane (15) est prévue du côté des microorganismes produisant de la cellulose, ladite membrane étant principalement imperméable pour les microorganismes et la cellulose, mais perniéable au gaz ou un à composant gazeux.

11. Procédé selon la revendication 10,
**caractérisé en ce que** la seconde membrane (15), forme avec la première membrane (4) un espace creux, dans lequel le corps de cellulose (7) grandit.

12. Disposition pour la fabrication d'un corps contenant de la cellulose utilisant des microorganismes produisant de la cellulose, lesquels sont alimentés par une solution nutritive, avec un récipient (1) doté d'une membrane (4), laquelle est principalement imperméable aux microorganismes et à la cellulose, mais est perméable à la solution nutritive,
**caractérisé en ce que** le récipient comporte d'un premier côté de la membrane (4) une chambre (2) pour la solution nutritive, et de l'autre côté de la membrane (4) une seconde chambre (3) avec une atmosphère gazeuse, dans laquelle des microorganismes produisant de la cellulose sont prévus.

13. Disposition selon la revendication 12,
**caractérisé en ce que** le récipient (1) comporte au moins une ouverture (5, 6 ; 8, 9) pour permettre un échange de gaz et/ou de solution nutritive.

14. Disposition selon la revendication 12 ou 13,
**caractérisé en ce que** le récipient (1) est fermé, à l'exception au moins d'une ouverture existante facultative (5, 6 ; 8, 9) permettant un échange de gaz et/ou de solution nutritive.

15. Disposition selon l'une quelconque des revendications 12 à 14,
**caractérisé en ce qu'**une seconde membrane (15) est prévue, laquelle forme avec la première membrane (4) un ou plusieurs espaces creux dans lesquels le corps de cellulose (7) grandit.
